# EUROPEAN PATENT SPECIFICATION

(11) **EP 1 165 057 B1**
(45) Date of publication and mention of the grant of the patent: **02.07.2008**
(21) Application number: 00919955.5
(22) Date of filing: 30.03.2000
(51) Int. Cl.: A61K 9/70, A61P 31/04, A61P 29/00

(54) **ENDODONTIC FIBERS AND METHODS OF USE THEREFOR**
ENDODONTISCHE FASERN UND VERFAHREN ZU IHRER VERWENDUNG
FIBRES ENDODONTIQUES ET LEURS METHODES D'UTILISATION

(30) Priority: 02.04.1999 US 127497 P
(43) Date of publication of application: 02.01.2002
(73) Proprietor: FORSYTH DENTAL INFIRMARY FOR CHILDREN, Boston Massachusetts 02115 (US); President and Fellows of Harvard College, Cambridge, MA 02138-5701 (US)
(72) Inventor: GILAD, Jack, Chestnut Hill, MA 02167 (US); STASHENKO, Philip, Norfolk, MA 02056 (US); GOODSON, Max, Cambridge, MA 02138 (US)
(74) Representative: Aston, Heidi Frances
(86) International application number: PCT/US2000/008582
(87) International publication number: WO 2000/059469

(56) References cited:
- EP-A- 0 140 766
- US-A- 5 114 718
- GILAD JZ, TELES R, GOODSON M, WHITE RR, STASHENKO: "Development of a clindamycin-impregnated fiber as an intracanal medication in endodontic therapy" JOURNAL OF ENDODONTICS, vol. 25, no. 11, 1999, pages 722-727, XP000971017
- GOODSON JM, HOLBOROW D: "Monolithic tetracycline-containing fibers for controlled delivery to periodontal pockets" JOURNAL OF PERIODONTOLOGY, vol. 54, no. 10, 1983, pages 575-579, XP000961292 cited in the application

## Description

### BACKGROUND OF THE INVENTION

Endodontics is a field of dentistry concerned with the biology and pathology of the dental pulp and periapical tissues. Endodontic treatment employs a set of techniques, such as chemomechanical debridement, irrigation, drainage of hard and soft tissue, trephination, and antimicrobial therapy, with the goal of avoiding the extraction of a damaged, infected or diseased tooth.

Normal vital pulp is sterile, and the role of bacterial infection in the pathogenesis of pulpal and periapical disease is well established. Infected or necrotic pulpal tissue renders the pulp chamber and root canal a potential reservoir of bacteria, and disinfection of the tooth is one of the primary justifications for the chemomechanical aspects of root canal therapy. Recent data demonstrate a high incidence of root canal failure in necrotic teeth treated in a single visit, attributed to bacteria remaining in complex anatomical spaces such as accessory canals, fins, deltas and isthmuses (Sjorgen et al., Int. Endo. J. 30:297-306 (1997)). Other studies have reported the ability of bacteria to migrate into dentinal tubules and survive therein (Nagaoka et al., J. Endodon. 21: 70-73 (1996)). It is speculated that the success rate of endodontic treatment could be 26% higher if the root canal is successfully disinfected prior to the final restoration (Sjorgen et al., Int. Endo. J. 30:297-306 (1997)).

Root canal infections are characterized as polymicrobial infections which tend to be dominated by anaerobic bacteria. As a group, the common endodontic microbes associated with treatment failure include *F. nucleatum, P. intermedia, P. micros, S. intermedius, P. endodontlis, P. gingivalis, P. melaninogenica, E. lentum, V. parvula, S. sanguis, P. buccae, P. oralis, and P. acnes.* (Haapasalo, FEMS Immunol. and Medical Micro 6:213-217 (1993) and Sundqvist, J. Endodon. 7:257-262 (1992)).

Post-operative periapical pain and interappointment flare-ups are also routinely attributed to the presence of bacteria, and/or their by-products, within the root canal. Typically, an initial bacterial infection triggers a host-mediated inflammatory response, the consequences of which underlie the flare-up patient's clinical symptoms. It has been reported that bacteria surviving instrumentation and irrigation proliferate rapidly in empty root canals (Bystrom and Sundqvist, Oral. Surg. Oral. Med Oral Pathol 55:307-312 (1983), and there is a positive correlation between the number of bacteria present in a root canal and the incidence of interappointment flare-ups. The presence of black-pigmented, gram negative anaerobes in the root canal usually accompanies patient complaints of pain, swelling, and tenderness to percussion (Haapasalo, FEMS Immunol. and Medical Micro 6:213-217 (1993)). Thus, the successful elimination of bacteria from root canals may lower the incidence of flare-ups.

Antibiotics have historically been used as an adjunct to endodontic treatment either by systemic or local administration. Currently, antibiotic treatment for root canal infections and exacerbations is limited to systemic administration. Thus, in light of the established correlations between the primary and secondary effects of bacterial presence and the incidence of both interappointment flare-ups and treatment failure, there is a clear need for an efficacious method of delivering and sustaining substantial concentrations of intracanal medicaments, particularly antibiotics.

During the 1950's a polyantibiotic paste (PBSC) was devised for use as an intracanal medicament (Grossman, L.I., J. Amer. Dent. Assoc. 43: 265-278 (1951). PBSC consisted of penicillin to target gram positive organisms, bacitracin for penicillin-resistant strains, streptomycin for gram negative organisms and caprylate sodium to target yeast, all suspended in a silicone vehicle. Although, clinical evaluation suggested that polyantibiotic paste conferred a therapeutic benefit (fewer treatments to achieve a negative culture), the composition was ineffective against anaerobic species (which are now appreciated as the dominant species responsible for treatment failure). In 1975 the Food and Drug Administration (FDA) banned PBSC for endodontic use primarily because of the risks of sensitization and allergic reactions attributed to the penicillin. This underscores the importance of improving historical endodontic methodologies, particularly local delivery methods, in light of contemporary knowledge and technological advances.

### SUMMARY OF THE INVENTION

The invention relates to the use of endodontic fibers as claimed in claim 1-9 comprising a biocompatible polymer vehicle which is permeable to medicaments, or combinations of medicaments, dispersed, e.g., homogeneously, therein. Such fibers are used, in a method for the local delivery and sustained release of medicaments to intracanal treatment sites. Endodontic fibers of this invention include modified periodontal fibers and intracanal fibers.

One embodiment of the invention relates to modified periodontal fibers suitable for delivery of medicaments to intracanal treatment sites. These first generation endodontic fibers, referred to herein as "modified peridontal fibers", represent an adaptation of an ethylene vinyl acetate delivery vehicle (see U.S. Patent No. 4. 764. 377 and U.S. Patent No. 4,892,736) previously developed to administer therapeutic agents during the course of periodontal treatment (Gilad, "Development of a Clindamycin Impregnated EVA fiber as an Intracanal Medicament in Endodontic Therapy," Master of Medical Sciences Thesis, Harvard University School of Dental Medicine, defended April 2, 1998). Specifically, the periodontal fibers have been modified to confer properties which allow the use of the fiber within an intracanal treatment site, e.g., to confer specific physical characteristics such as form and consistency. In one embodiment, the modification comprises the treatment of the periodontal fiber with an agent such as a biocompatible refrigerant spray (e.g., Endo Ice).

In an alternative embodiment the invention also relates to a second generation endodontic fiber, referred to herein as an "intracanal fiber", which can be specifically designed for use in intracanal delivery methods, thereby obviating the need to modify a peridontal fiber for use in intracanal sites. Such design can include an alteration in the composition and/or ratio of components of the fiber.

The invention is demonstrated herein using clindamycin\ethylene vinyl acetate (EVA) fibers; however, this example is not intended to limit the scope of the invention in any way. For example, the contemplated intracanal fiber can be formulated to have a polymeric composition, surface tackiness, stiffness, glass transition temperature, and/or diameter selected to confer characteristics compatible with placement within the root canal.

In addition, the choice of medicament and the dose at which it is incorporated into the disclosed endodontic fibers (e.g., modified peridontal fibers) are optimized to produce a fiber that is most likely to achieve the desired therapeutic effect. The intracanal fibers exemplified and contemplated herein are ideally suited for the local delivery and sustained release of intracanal medicaments and thus enable numerous intracanal delivery methods.

In one aspect of endodontic use, endodontic fibers (e.g., modified peridontal fibers or intracanal fibers) are utilized for the intracanal delivery and sustained release of antibiotics predicted to be efficacious for the treatment of an established endodontic bacterial infection. The goal of the intracanal delivery of antibiotics in this context is to achieve a sufficient drug concentration and duration of contact, to effect inhibition (e.g., partial or complete inhibition) of all bacterial growth within the pulp chamber and root canal, thereby obviating the need for systemic antibiotic administration. Ultimately, the ability to successfully treat established bacterial infections will reduce endodontic treatment failures.

In an alternative embodiment, an intracanal delivery method using endodontic fibers of the invention is utilized prophylactically to disinfect a root canal receiving endodontic treatment prior to the application of a final restoration. In this context, the local delivery method is employed to eradicate any residual bacteria which were not removed by the chemomechanical preparation of the canal. More specifically, the purpose of this method of delivery is to suppress bacterial growth, particularly the proliferation of black-pigmented, gram negative organisms within the root canal. Such prophylaxis can reduce the level of patient pain due to inflammation and the occurrence of interappointment flare-ups, and ultimately minimize the risk of treatment failures.

In other embodiments of the invention, endodontic fibers described herein can be used to deliver alternative intracanal medicaments necessitated by a course of endodontic treatment. For example, in an effort to attenuate a host-mediated inflammatory response resulting from the presence of bacterial by-products in periapical tissues, an anti-inflammatory agent, either alone or in combination with an antibiotic, can be incorporated into the endodontic fiber.

### BRIEF DESCRIPTION OF THE FIGURES

Figure 1 is a bar graph indicating the number of colony forming units (CFUs) isolated from paper point or crushed teeth samples of extracted human teeth infected *in vitro* with *P. intermedia* and treated with either clindamycin/EVA fibers, or EVA control fibers.
Figures 2A-2C are bar graphs indicating the CFUs isolated from extracted human teeth infected *in vitro* with a mixed inoculum of *F*. *nucleatum, P. micros,* and *P. intermedia,* respectively, and treated with either clindamycin/EVA fibers or EVA control fibers. The graphs summarize clindamycin activity against each of the three species of bacteria present in the inoculum.
Figures 3A and 3B are bar graphs summarizing the CFU load recovered from auto-infected ferret canine teeth undergoing root canal therapy, treated with either clindamycin/EVA fibers or EVA control fibers.

### DETAILED DESCRIPTION OF THE INVENTION

The role of endogenous microflora as a source of bacterial infection contributing to endodontic treatment failure is well established (Kakehashi, S. et al., Oral Surg. 20: 340-348 (1965)). The bacterial species most often associated with infections of endodontic origin belong to the genera *Prevotella, Porphyromonas, Fusobacterium, Peptostreptococcus, Eubacterium* and *Streptococcus.* Some published studies have implicated species of black-pigmented, gram negative anaerobes as possible endopathogens (based on a frequency of isolation in the 25% to 50% range from teeth experiencing treatment failure), however, no single species has been proven to be more pathogenic than others (USAIDR Information Bulletin, 4(3) 1990).

A "flare-up" is defined as pain and/or swelling which occurs within a few hours to a few days after a root canal treatment procedure. Depending upon the severity of the symptoms, there is often a sufficient disruption of the patient's lifestyle such that the patient initiates an unscheduled visit and treatment. Published studies suggest that the presence of members of the black-pigmented Porphyromonas spp. (particularly *Porphyromonas gingivalis* and *Porphyromonas endodontalis)* within the root canal correlate with the type of acute symptoms responsible for interappointment flare-ups (Yoshida et al., J. Endodon. 13:24-28 (1987)). Thus, in addition to reducing the failure rate of endodontic treatment, it also desirable to reduce the frequency of interappointment flare-ups.

Antibiotics have historically been used as an adjunct to endodontic treatment, either by systemic or local administration. Currently, antibiotic treatment for root canal infections and exacerbations is limited to systemic administration. Commonly prescribed antibiotics include penicillins (e.g., penicillin V, amoxicillin), erythromycins (e.g., erythromycin stearate), lincosamides (e.g., clindamycin) and cephalosporins (e.g., cephalexin). The decision to use antibiotics is often made by the practitioner in relation to his or her own treatment philosophy. The choice is made in light of the knowledge that systemic antibiotics should be prescribed with restraint because of the possibilities of hypersensitivity or anaphylactic reactions, toxicity, adverse systemic effects, and the development of resistant strains of microorganisms.

A critical reevaluation of the merits of delivery devices, vehicles, techniques, and medicaments which have been historically utilized for intracanal delivery methods reveals that the use of intracanal medicaments in general, and in particular the use of intracanal antibiotics, has been criticized for inadequate spectrum of activity and short duration of effectiveness. The former issue has been addressed by improved microbiological sampling techniques, particularly anaerobic culturing techniques, which now provide practitioners with an accurate profile of the bacterial species associated with endodontic infections. This information enables practitioners to prescribe more appropriate antimicrobial agents. As a result, the short duration of effectiveness emerged as the major flaw of intracanal delivery protocols. The endodontic fibers described herein address this issue by allowing a treatment strategy which is demonstrated to be capable of the sustained release of active medicament for at least 14 days *(in vitro).*

The disclosed endodontic fibers enable a delivery system and method capable of the sustained release of any class of medicament that is necessitated as a consequence of therapy, particularly root canal therapy. In preferred embodiments, the invention provides a therapeutic method for the treatment of an endodontic bacterial infection, or alternatively a controlled aseptic technique suitable for use as an adjunct to conventional chemomechanical debridement and irrigation techniques.

Systemic administration relies upon circulatory elements to bring active drug to an infected site. However, it is well recognized that infected and/or inflamed periradicular tissue and necrotic pulpless teeth do not possess a normal vasculature. This practical consideration renders systemic administration inefficient, particularly when it is combined with the knowledge that in order to be effective, an antibiotic must be in contact with the targeted microorganisms. These facts clearly compromise the potential utility of systemically administered prophylactic antibiotics.

In contrast, a local delivery strategy confers the therapeutic benefit of delivering a medicament directly to the targeted tissue space. In addition, use of the disclosed delivery vehicle and method is readily amenable to both bacteriological sampling and sensitivity screening in the event that an infection does not respond to an initial course of treatment, and the easy removal of the fiber in the case of an unforeseen complication or allergic reaction. The latter feature represents a significant improvement over the historical use of paste or liquid compositions.

Furthermore, the ability to establish substantial local concentrations of an antibacterial agent also minimizes the risk of contributing to the development of drug resistant pathogens. One of the major contraindications to the use of systemic antibiotics is the theoretical possibility that bacteria not affected by the relatively low concentrations achieved by oral administration will give rise to strains having multiple drug resistance. Intracanal delivery also spares the patient from unwanted side-effects commonly associated with systemic administration strategies. For example, systemic clindamycin administration has been associated with the occurrence of pseudomembranous colitis, a sufficiently deleterious side effect to account for the reluctance of many clinicians to prescribe clindamycin, despite its broad spectrum of activity. However, given the dosages required to cause pseudomembranous colitis, along with the requirement for gastrointestinal contact, it is unlikely that the intracanal use of clindamycin/EVA endodontic fibers would trigger such an adverse side effect. This later benefit can be decisive in terms of prescribing a particular medicament whose spectrum of activity may be well suited for the task, but systemic administration carries a high risk of toxicity.

The intracanal fibers described herein are specifically designed for use in intracanal delivery methods. The optimal composition of the fibers can be empirically determined to confer the physical characteristics and polymeric composition required for intracanal use. In a preferred embodiment, the endodontic fiber has a diameter which facilitates placement deep within the cleaned and reshaped root canal, e.g., a diameter of from about 0.1 to about 0.5 mm. The composition and glass transition temperature of the polymer can also be selected to confer surface characteristics and a level of rigidity required to accomplish the aseptic placement of the fiber within the root canal, and to facilitate the subsequent conformity of the fiber to the contours of the root canal. Intracanal fibers suitable for use in the disclosed invention can further comprise additional features characteristic of the modified periodontal fibers exemplified herein. More specifically, particularly preferred fibers are characterized by additional features such as being odorless, being colorless, permitting deep penetration of the root canal, being suitable for use with a variety of therapeutic agents, being capable of the sustained release of at least one active agent (e.g., for at least a one week period of time *(in vitro*))*,* and not staining tooth structure or interfering with standard bacterial culture techniques.

Biocompatible vehicles useful for the formulation of the disclosed endodontic fibers are biocompatible synthetic or natural copolymers, which may or may not be biodegradable. For example, polymers such as collagen, cellulosic polymers, glycolic acid polymers, methacrylate polymers, ethylene vinyl acetate polymers, ethylene vinyl alcohol copolymers, polycaprolactone, polyurethanes and polylactides and combinations thereof are suitable for use in this invention. The form (i.e., shape) of the polymer composition is not critical as long as the form allows the composition to be positioned within the root canal. In a preferred embodiment the form of the polymer composition is a string or fiber. For example, polymers useful for the preparation of second generation intracanal endodontic fiber according to the invention contemplated herein are described in U.S. Patent No. 4, 764, 377 and U.S. Patent No. 4,892,736.

It is recognized that in the preparation of an endodontic fiber for use in an intracanal delivery method, certain inert substances may be included to further modify the delivery characteristics, or to serve as carriers of the active agent, including solvents, suspending agents, surfactants, viscosity-controlling agents, and other pharmaceutically acceptable materials which may be desirable to solubilize or stabilize the therapeutic agent (or agents) in the delivery vehicle, or to control the rate of permeation or the action of the agents after permeation.

According to the invention, the modified periodontal fiber or intracanal fiber is impregnated with one or more medicaments using methods known in the art. A wide variety of medicaments may be used in the invention, either alone or in combination. Therapeutic agents suitable for use in the invention include, but are not limited to: antibiotics such as clindamycin, tetracycline, neomycin, kanamycin, metranidazole or canamycin; antimicrobial agents such as iodine, sulfonamides, mercurials, bisbiguanidines, or phenolics; anti-inflammatory agents such as indomethacin or hydrocortisone; immune reagents such an immunoglobulins, or antigen binding fragments of immunoglobulins or immunomodulatory agents such as methotrexate.

In addition, it is recognized that in certain forms of therapy, combinations of these agents in the same delivery vehicle may be utilized in order to obtain an optimal effect. Thus, for example, an antibiotic and an anti-inflammatory agent may be combined in the preparation of a single endodontic fiber, which could be used either as an adjunct or a substitute for traditional endodontic treatment protocols.

The choice of medicament, and the dose at which it is incorporated into the endodontic fiber, can be selected to produce fibers that achieve the desired therapeutic effect, in light of a particular set of factors. For example, the initial selection of an appropriate antibiotic, and the dose at which incorporated into the endodontic fiber, are empirical choices guided by knowledge of the bacterial species commonly associated with treatment failure, the condition of the particular tooth receiving treatment, and the time span between scheduled appointments. Properties desirable in an ideal intracanal antibiotic or antimicrobial agent (or combination thereof) for use during root canal treatment are that the medicament be germicidal to all, or at least a portion of, organisms present at the treatment site, rapidly effective, capable of deep penetration into the canal system, effective in the presence of organic matter, noninjurious to periapical tissues, chemically stable, odorless, tasteless, and inexpensive. In practice, the selection of a therapeutic agent for use in the described intracanal delivery methods will be dictated by the permeability of the delivery vehicle to the agent, the dose at which the agent can be incorporated into the fiber, and the toxicity of the agent.

For example, clindamycin is effective against many of the bacterial taxa commonly associated with endodontic treatment, and depending on the effective concentration achieved at the site of infection it can be either a bacteriostatic or a bacteriocidal antibiotic. It is effective against: *Actinomyces, Eubacterium, Fusobacterium, Propionibacterium,* microareophilic *Streptococci, Peptococcus, Peptostreptococcus, Veillonella, Prevotella,* and *Porphyromona.* Also, hypersensitivity and anaphylaxis as a result of clindamycin exposure is extremely rare. In addition, data presented herein demonstrates that clindamycin/EVA fibers are active *in vitro* against the black-pigmented *Prevotella* and *Porphyromonas* species, which are commonly associated with the occurrence of flare-ups. Clindamycin binds exclusively to the 50S subunit of bacterial ribosomes and interferes with peptidyl transfer, which prevents elongation of peptide chains and ultimately suppresses bacterial protein synthesis. (AHFS Drug Information Reference, 388-393 (1997). The minimum inhibitory concentration (MIC) of clindamycin for most susceptible aerobic and microaerophilic bacteria is 0.1-0.4 mg/ml, and is often observed to be much lower than the corresponding penicillin or erythromycin MIC. Published studies indicate that over the twenty year period ending in 1986, there has been no marked change in the sensitivity of bacteria to lincomycins, and more specifically that 70% to 80% of all bacterial species isolated have remained susceptible to clindamycin. Conversely, a reduction in bacterial sensitivity, and in some instances evidence of resistance, has emerged among amoxicillin, cephalosporins, and erythromycin during the same period of time (Woods, Aust. Dent. J. 33:420-423, 505-510 (1988)).

The use of the disclosed endodontic fibers and methods during the course of - endodontic treatment can readily be adapted to complement a typical endodontic treatment program. Conventional root canal therapy is performed over a series of visits, to allow sufficient time to pass from the initial pulpectomy, chemomechanical debridement, and irrigation to ensure that the pulp chamber and root canal are aseptic prior to the application of the final restoration. Therefore, the anticipated use of the medicament-impregnated fiber in the context of either a prophylactic method, or for the treatment of an established infection, does not require, but could optionally utilize, a biodegradable polymer. The controlled release characteristic of the fiber, combined with the opportunity for periodic replacement, makes the method compatible with conventional endodontic treatment protocols, and increases the likelihood that the local administration will achieve its desired therapeutic effect.

The modified periodontal fiber and intracanal fiber claimed and described herein are suitable for use in any and all of the disclosed methods of intracanal delivery, including, but not limited to, prophylactic disinfection of the root canal, treatment of a bacterial infection, attenuation of a host-mediated inflammatory response, and the sustained delivery of an appropriate intracanal medicament necessitated by endodontic treatment.

The invention will now be further described by the following non-limiting examples.

### EXAMPLES

### Preparation of a Clindamycin/EVA Endodontic Fiber and in vitro Efficacy Against Endodontic Pathogens

First generation (periodontal) clindamycin/EVA endodontic fibers were prepared according to a method used for the preparation of periodontal tetracycline/EVA fibers (as described in U.S. Patent 4,892,736) with slight modifications. In brief, 0.075 g calcium phosphate monobasic (CaH₂PO₄) was combined with 10 ml distilled water (dH₂O) and added to a solution consisting of 0.050 g clindamycin phosphate and 10 ml dH₂O. The combined solution was then lyophilized for 24 hours. The resultant powder was filtered through a #325 mesh sieve (W.S. Tyler Co., Mentor, OH) in order to achieve uniform particle size of 45µ. The resultant yield (125 mg) was combined with 375 mg of EVA particles (USi Inc., TN) and processed through an extrusion plastometer (Tinius Olsen Co.) at diameters of 2.0 mm, 1.0 mm, and 0.5 mm. The final extrusion produced a 250 mm long fiber, with a calculated approximate dose of 2.0 mg clindamycin/10 mm fiber.

Bacterial sensitivity to the modified peridontal clindamycin/EVA fiber was determined by placing 10 mm long fiber segments of on blood agar plates colonized by the following bacterial species: *F. nucleatum* (ATCC 364), *P. intermedia* (ATCC 25621), *P. micros* (ATCC JH20), *S. intermedius* (ATCC 27335), *P*. *endodontalis* (ATCC 35406), *P. gingivalis* (ATCC 381), *P. melaninogenica* (ATCC 25845), *E. lentum* (ATCC 25559), *V. parvula* (ATCC 10790), *S. sanguis* (ATCC 551), *P. buccae* (ATCC 33574), P. oralis (ATCC 33269), or *P. acnes* (ATCC 11827). Assay plates were incubated in anaerobic chambers for four days prior to the measurement using a millimeter ruler and recording of the resulting zones of inhibition. Table 1 summarizes the observed zones of inhibition. Control fibers, consisting of EVA fibers without clindamycin, failed to produce any detectable growth inhibition of the above-listed bacterial species. These data demonstrate that clindamycin/EVA fibers have significant antimicrobial activity against endodontic microorganisms.

**TABLE 1**

| Assessment of Fiber Effectiveness Versus Endodontic Bacteria *in Vitro* | |
|---|---|
| Bacteria | Zone of inhibition (mm) |
| *P. cingivalis* | >100 |
| *P. intermedia* | >100 |
| *P. buccae* | 45 |
| *P. oralis* | *45* |
| *S. sanguis* | 35 |
| *P. acnes* | 30 |
| *V. parvula* | 30 |
| *F. nucleatum* | 25 |
| *P. melaninogenica* | 25 |
| *E. lentum* | 18 |
| *S. intermedius* | 15 |
| *P. micros* | 10 |
| *P. endodontalis* | 10 |

*In vitro* Suppression of Bacterial Growth in Extracted Human Teeth Infected with Endodontic Pathogens by Clindamycin/EVA Fibers

In order to test the efficacy of the clindamycin/EVA fibers an *in vitro* assay was developed based on the use of extracted human teeth which are persistently infected with endodontic pathogens. Thirty-two extracted human teeth (anteriors/premolars) were sectioned at the cementoenamel junction, shaped with 0.04 taper nickel-titanium rotary endodontic instruments (Tulsa Dental, Tulsa, OK.) and fully cleaned with 5.25% sodium hypochlorite (NaOCI), followed by thorough flushing with distilled water. The smear layer was not removed. The teeth were then sterilized by autoclaving for 20 minutes and coated with sticky wax, leaving the apical foramen and coronal orifice patent.

In initial studies *P*. *intermedia* was grown on blood agar plates and was used as the infecting microorganism. BBL Mycoplasma broth was prepared by sterilizing a solution consisting of 1.05g of BBL mycoplasma broth powder with 0.1g glucose, 0.5 g hemin, 50 ml dH₂O and 0.025 g L-cysteine (at pH 7.4-7.6). Under nitrogen influx, tubes of sterile broth were combined with *P. intermedia* colonies from the stock agar plates, and diluted to a concentration of 10⁹ bacteria/ml (optical density reading of 1.0 @ 600 nm), and then diluted to 10⁶ bacteria/ml by adding 50 µl of bacterial broth to 450 µl sterile media. The sterile teeth were then introduced into tissue culture wells filled with the bacterial broth. The wells were lightly covered and placed in an anaerobic chamber for four days.

For sampling purposes, the teeth were moved into dry wells, and sterile paper points were inserted for 15 seconds. The resulting sample was then dispersed by vortexing to release the sample microorganisms into 1 ml aliquots of sterile media. Ten-fold serial dilutions were performed by transferring 50 µl of bacterial broth into 450 µl of sterile media under nitrogen influx for 10⁻², 10⁻³ and 10⁻⁴ concentrations. 100 µl samples were spread onto blood agar plates, and cultured for four days under anaerobic conditions. On the fifth day the teeth were transferred into a second plate containing 500 µl of fresh media and returned to anaerobic culture conditions for an additional 4 days. After eight days, paper point sample plates corresponding to teeth exhibiting positive growth were quantified for number of colony forming units (CFUs) and two teeth had to be eliminated from the study due to lack of bacterial colonization.
Seven colonized teeth were randomly selected for use as control teeth (receiving 10 mm long EVA fibers formulated without any antibiotic), and seven other infected teeth were utilized as experimental teeth (receiving 10 mm long clindamycin/EVA fibers ). Periodontal clindamycin/EVA fibers were prepared as described above, and to facilitate fiber manipulation and insertion they were treated to decrease their surface tackiness and increase their rigidity by spraying with a biocompatible refrigerant spray (Endo Ice,) (thereby producing "modified periodontal fibers").

Following fiber placement the teeth were placed into new wells with 500 µl of fresh sterile media and returned to anaerobic culture conditions. Wells were replenished with fresh media daily for four days, at which point the teeth were sampled with paper points and assayed for bacterial colonization according to the serial dilution and plating method described above. In order to ensure the detection of bacterial species colonizing locations which are theoretically not accessible paper point sampling (such as complex anatomical spaces in the root canal system, or dentinal tubules) entire teeth were individually fractured and crushed in sterile autoclave bags and then dispersed into culture tubes containing 1 ml sterile media. Crushed-tooth samples were serially diluted and plated according to the method described above for the paper point samples. CFUs from the suitably diluted blood agar plates were quantified 7 days later. Colony identification was verified by morphometric analysis. Statistical analysis of the differences observed between control (EVA fibers without antibiotic) and experimental (clindamycin/EVA fibers) were evaluated with the non-parametic Wilcoxin Rank Sum Test.

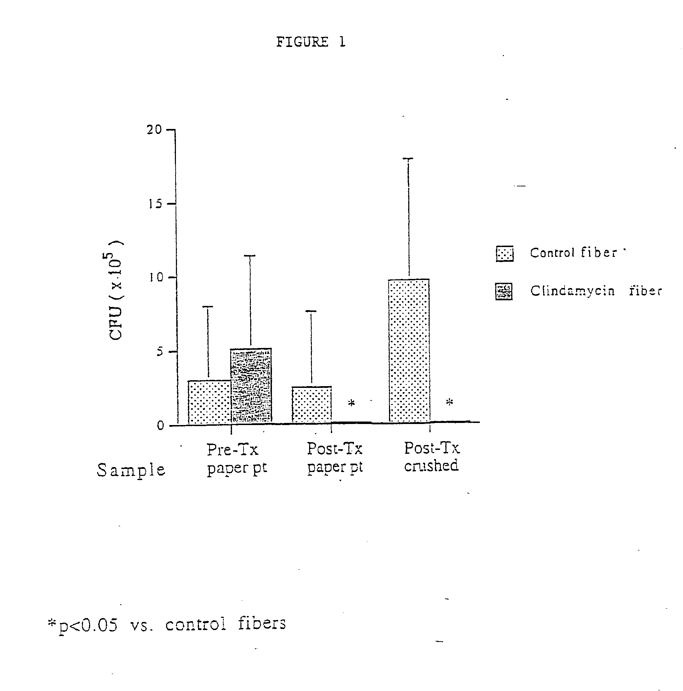

The data in Figure 1 demonstrate that clindamycin/EVA fibers are efficacious in suppressing bacterial growth in teeth infected with *P. intermedia.* All seven experimental teeth treated with clindamycin fibers demonstrated no growth from either paper point or crushed tooth samples, demonstrating the efficacy of the fiber. In contrast, six of seven control teeth that received EVA fibers demonstrated positive growth. Statistical analysis utilizing the Wilcoxin rank sum test revealed that there were no differences between baseline infected teeth with respect to CFU quantification prior to fiber placement (p>0.05). Samples from both paper point and crushed experimental teeth treated with clindamycin/EVA were significantly different from control teeth (p<0.05).

In a second experiment, the ability of clindamycin/EVA fibers to reduce bacterial infection with a mixed inoculum was assessed. Sixteen new teeth were prepared as described above, and placed into tissue culture wells containing 350 µl sterile media, and a mixture of 50 µl *F*. *nucleatum,* 50 µl *P. micros* and 50 µl *P. intermedia* at a concentration of 10⁹ bacteria/ml. Paper point samples were once again taken after four days to confirm and quantify bacterial growth. Modified peridontal fibers, prepared according to the method described above, were placed in 16 teeth for four days (eight controls and eight experimental), and the teeth were transferred into wells of fresh media daily. After obtaining paper point samples the teeth were crushed and serially diluted samples were prepared, and plated as described above.

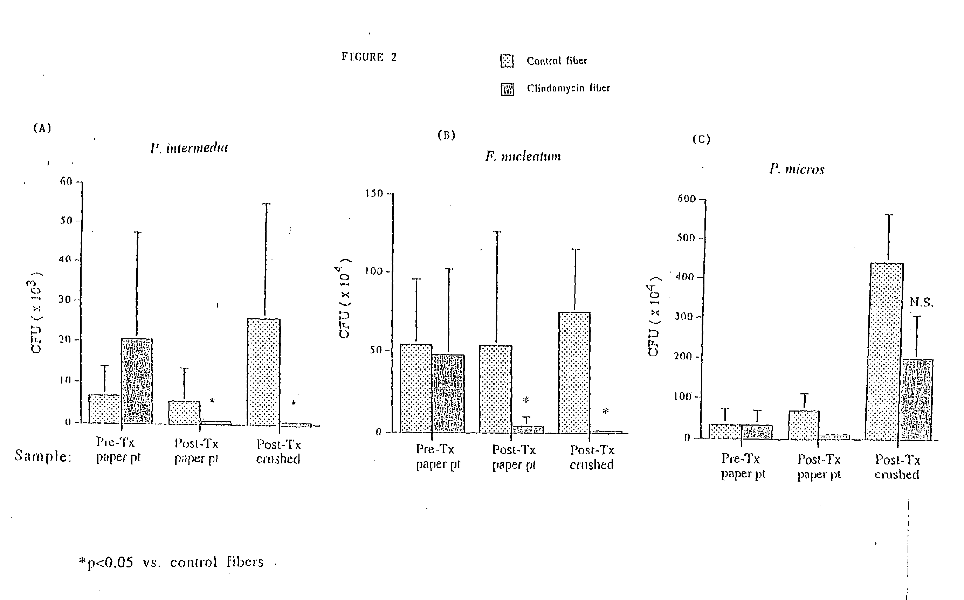

One week following fiber placement, the CFU load was quantified and compared with the baseline CFU counts. Colony identification was again verified by morphometric analysis. Wilcoxian rank sum tests revealed that the differences between control and experimental teeth were all statistically significant (p<0.05). When comparing individual teeth for pre-treatment and post- treatment CFU values by t-Test. *F*. *nucleatum* groups were significantly different (p<0.05) as were *P*. *intermedia* groups (p:<0.05). However, the differences between the *P. micros* control and experimental groups were not significant (p>0.05). Thus, as shown in Figure 2. the intracanal delivery of clindamycin was most effective against *P*. *intermedia,* less effective against *F. nucleatum,* and apparently ineffective against *P. micros* in the context of a mixed inoculum, despite the fact that clindamycin inhibits the growth of pure cultures of *P. micros* on blood agar plates.

### In vivo Suppression of Bacterial Growth in Auto-Infected Root Canals of Ferret Canine Teeth by Clindamycin/EVA Fibers

### Experiment 1

Ferret canine teeth have been utilized successfully in endodontic research to study the induction of periapical lesions (Fouad, Endo. and Dent Trauma 8:56-62 (1992)), and are long and large enough to accommodate endodontic fiber placement. Therefore, ferret canine teeth provide an *in vivo* model system in which to evaluate the ability of clindamycin endodontic fibers to inhibit bacterial growth in auto-infected root canals. Eight male ferrets (12 weeks old, approximately 3 lbs each, Marshall Farms, Rose, NY.) were utilized to evaluate the *in vivo* efficacy of clindamycin/EVA fibers. Ferrets were premedicated with atropine (0.04 mg/kg, subcutaneously) 30 minutes prior to the procedure. Animals were then anesthetized intramuscularly with ketamine HCl (30 mg/kg) and xylazine (3 mg/kg) in sterile PBS. This was supplemented with a repeat dose of ketamine and xylazine when necessary. The pulp cavity of thirty two ferret canine teeth (4 teeth per each of 8 animals) was surgically exposed (using a #2 round bur and a high speed handpiece). Working lengths were confirmed radiographically. The root canal system was instrumented with 0.04 taper nickel-titanium rotary endodontic instruments to approximately a 0.30 mm apical preparation and irrigated with sterile saline. The teeth were left open for seven days to allow for bacterial colonization from the oral cavity. The auto-colonized teeth were subsequently closed with a cotton pellet and Intermediate Restorative Material (IRM) for a period of 14 days to allow for anaerobic bacterial growth and the development of pathogenesis. The teeth were then reopened (under general anesthesia) for modified peridontal fiber placement. Four of the eight animals received experimental clindamycin/EVA fibers (four teeth/animal) and the remaining four animals received negative control/EVA fibers. The teeth were resealed with cotton and IRM.

Treatment efficacy was determined seven days later after preparing crushed tooth samples from the treated animals. Briefly, the animals were placed under anesthesia, their mouths were swabbed with iodine and alcohol, the teeth were extracted intact and surface sterilized with iodine and alcohol, before being crushed inside of sterile autoclave bags. The fibers were subsequently removed, and the tooth fragments were placed into 1 ml of prereduced anaerobically-sterilized (PRAS) transport medium under nitrogen influx, and immediately transported to the laboratory for serial dilution and plating under nitrogen influx as described in the *in vitro* studies above. Although the ferret canine teeth were generally long enough to accept and accommodate the fibers, they were sufficiently curved to complicate the extraction process. As a result some of the teeth were fractured and had to be excluded from the study.

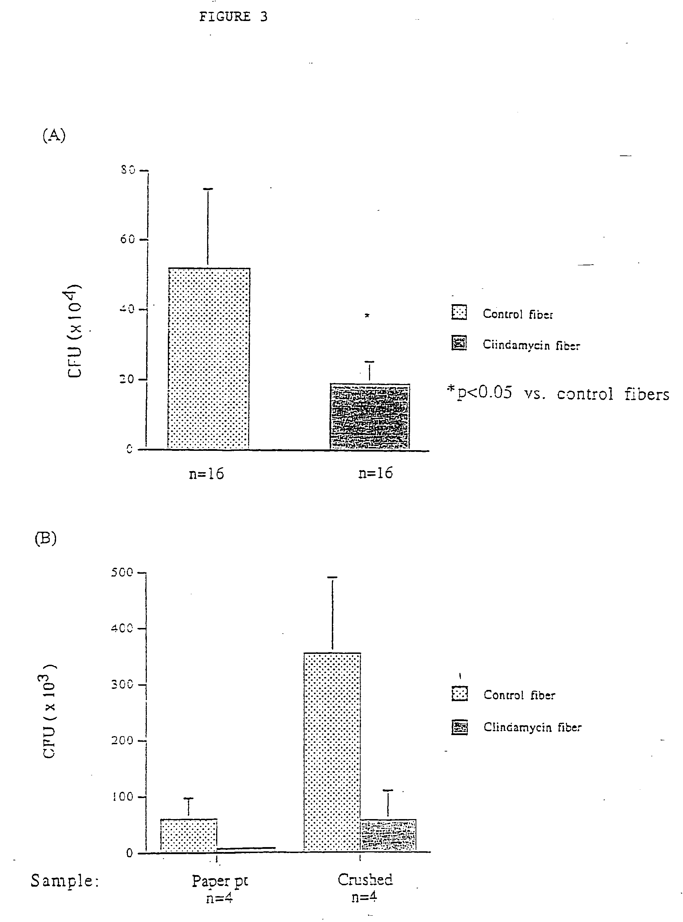

Samples were serially diluted and incubated on blood agar plates in an anaerobic environment for seven days, and CFU's were quantified. Resultant data are presented in Figure 3A. The average CFU count observed for control teeth (EVA fibers only) is 5.19 x 10⁵ CFU's compared to an average of 1.89 x 10⁵ CFU's for the experimental teeth (clindamycin/EVA fibers). This represented a 2.75-fold decrease in CFU load in the clindamycin treated teeth. The Wilcoxin rank sum test confirmed that the differences between control and experimental groups were statistically significant (p<0.05).

### Experiment 2

In a second experiment two ferrets (one control and one experimental animal) were utilized. The control animal had four teeth that treated with modified peridontal EVA fibers that contained no clindamycin and a fifth tooth which was neither accessed nor instrumented, but which was ultimately extracted, crushed, and sampled. The experimental animal had three teeth which received modified peridontal clindamycin/EVA fibers and a fourth tooth that was treated with a modified peridontal tetracycline/EVA fiber. Paper point and crushed tooth samples were prepared as described above; however, two teeth were fractured upon extraction, and were not included in the data set.

The resultant data are summarized in Figure 3B. Statistical analysis for this small sample was not employed. Nonetheless, the experimental teeth showed a 6.3-fold decrease in CFU load when quantified from paper point samples, and a 4.3-fold decrease from crushed samples when compared with controls. The tetracycline/EVA fiber appeared to possess similar efficacy to that of the clindamycin fiber. The unaccessed control tooth showed no bacterial growth, establishing that the tooth isolation technique did not result in any contamination from the external root surface.

DNA-DNA hybridization checkerboard analysis as described by Socransky et al., Biotechniques 17: 788-792 (1994), was performed, using samples harvested from blood agar plates following one week of growth from both of the above *in vivo* experiments. The proportion of teeth colonized with each bacterial taxa was evaluated (presence vs. absence). Summary data comparing control (EVA fiber only) versus experimental (clindamycin/EVA- or tetracline/EV A-treated) teeth are shown in Tables 2-4. The data indicate that the treatment of teeth with clindamycin/EVA fibers does not favor the development of a unique bacterial profile relative to the profile observed in control teeth receiving control fibers.

**Table 2**

| **Bacterial species in Control versus Clindamycin-treated ferret teeth by DNA checkerboard analysis (Crushed-tooth samples)** | | |
|---|---|---|
| | **Fiber Treatment** | |
| **Bacteria Present** | **Control (n-16)** | **Clindamycin (n=16)** |
| *A. naeslundii 1* | 16^{a} | 16 |
| *E. saburreum* | 16 | 14 |
| *P.micros* | 10 | 3 |
| *A naeslundii 2* | 16 | 16 |
| *S. anginosus* | 16 | 14 |
| *A. gerensceriae* | 16 | 16 |
| *A. odontolyticus* | 0 | 11 |
| *F. nucl ss polymorph* | 1 | 0 |
| *F. periodonticum* | 1 | 8 |
| *F. nucl ss nucleatum* | 3 | 7 |
| *P. acnes* | 0 | 2 |
| *L. buccalis* | 16 | 16 |

| | | |
|---|---|---|
| ^{a}number of positive samples | | |

**Table 3**

| **Bacterial Species in Control versus Clindamycin-treated ferret teeth by DNA checkerboard analysis (Experiment 2: paper point samples)** | | | |
|---|---|---|---|
| | | **Fiber treatment** | |
| **Bacteria Present** | **Control (n=4)** | **Clindamycin (n=3)** | **Actistite (n=1)** |
| *A. naeslundii 1* | 4^{a} | 3 | 0 |
| *A. actinom* | 4 | 3 | 0 |
| *E. saburreum* | 4 | 3 | 0 |
| *P. micros* | 4 | 3 | 0 |
| *A. naeslundii 2* | 4 | 3 | 0 |
| *A. gereneseriae* | 3 | 3 | 0 |
| *A. israelii* | 4 | 3 | 0 |
| *T. denticola* | 0 | 1 | 0 |
| *F. nucl ss polymorpyh* | 2 | 3 | 0 |
| *F. periodotiticum* | 2 | 3 | 0 |
| *N. mucosa* | 4 | 3 | 0 |
| *F. nucl ss nucleatum* | 4 | 3 | 0 |
| *E. corrodens* | 4 | 3 | 0 |
| *C. sputigena* | 3 | 3 | 0 |
| *L. buccalis* | 4 | 3 | 0 |

| | | | |
|---|---|---|---|
| ^{a}number of postitive samples | | | |

**Table 4**

| **Bacterial Species in Control versus Clindamycin-treated ferret teeth by DNA checkerboard analysis (Experiment 2: crushed-tooth samples)** | | | |
|---|---|---|---|
| | | **Fiber treatment** | |
| **Bacteria Present** | **Control (n=4)** | **Clindamycin (n=3)** | **Actistite (n=1)** |
| *A. naeslundii 1* | 2^{a} | 3 | 1 |
| *A. actinom* | 2 | 3 | 1 |
| *E. saburreum* | 1 | 3 | 1 |
| *P. micros* | 2 | 3 | 1 |
| *A. naeslundii 2* | 2 | 3 | 1 |
| *A. gereneseriae* | 2 | 3 | 0 |
| *A. israelii* | 2 | 3 | 1 |
| *T. denticola* | 1 | 1 | 0 |
| *F. nucl ss polymorpyh* | 2 | 3 | 1 |
| *F. periodonticum* | 1 | 3 | 0 |
| *N. mucosa* | 2 | 3 | 1 |
| *F. nucl ss nucleatum* | 2 | 3 | 1 |
| *E. corrodens* | 2 | 3 | 1 |
| *C. sputigena* | 1 | 3 | 1 |
| *L. buccalis* | 2 | 3 | 1 |

## Claims

1. Use of one or more medicaments in the manufacture of an endodontic fiber for the local delivery and sustained release of said one or more medicaments at an intracanal treatment site, wherein the endodontic fiber comprises a copolymer vehicle having incorporated therein the said one or more medicaments.

2. The use according to claim 1 wherein the endodontic fibre is for use in a method of treatment comprising :
a) positioning the fibre in the root canal such that the fibre is in direct contact with the treatment site; and
b) maintaining the fibre at the treatment site,
whereby said medicament is delivered to the treatment site at a controlled rate.

3. The use according to claim 1 wherein the endodontic fibre is for use in a method of treatment comprising :
a) inserting the fibre into a debrided and irrigated root canal such that the fibre is in direct contact with the treatment site; and
b) maintaining the fibre at the treatment site,
whereby the medicament is administered to the treatment site at a controlled rate.

4. The use according to claim 3, wherein the medicament is an anti-inflammatory agent, and said method of treatment involves in step (a) positioning the fibre into a debrided and irrigated root canal such that the fibre is in direct contact with the inflamed tissue; whereby the anti-inflammatory agent is delivered to the site of inflammation.

5. The use according to any one of claims 1 to 4, wherein the endodontic fiber comprises an ethylene vinyl acetate copolymer or wherein the endodontic fibre has a diameter of 0.1 to 0.5 mm.

6. The use according to any one of claims 1 to 3, wherein the said one or more medicaments are selected from the group consisting of antibiotics, antimicrobial agents, anti-inflammatory agents, immune reagents and antigen binding fragments.

7. The use according to any one of claims 1 to 3, wherein said one or more medicaments comprise one or more antibiotics selected from clindamycin, tetracycline, neomycin, kanamycin, metranidazole and canamycin.

8. The use according to any one of claims 1 to 3, wherein the fibre is an ethylene vinyl acetate copolymer having a diameter of 0. 1 to 2.0 mm and wherein said medicament is clindamycin incorporated at a dose of 2.0 mg to 5.0 mg per 10 mm of fibre.

9. The use according to any one of claims 1 to 3, wherein said copolymer vehicle is treated with a biocompatible refrigerant spray.

## Patentansprüche

1. Verwendung eines oder mehrerer Arzneimittel bei der Herstellung einer endodontischen Faser zur örtlichen Zuführung und Dauerfreisetzung des einen oder der mehreren Arzneimittel an einer Behandlungsstelle innerhalb des Kanals, wobei die endodontische Faser ein Copolymer-Vehikulum, in das das eine oder die mehreren Arzneimittel inkorporiert sind, aufweist.

2. Verwendung nach Anspruch 1, bei der die endodontische Faser zur Verwendung in einem Behandlungsverfahren ist, das aufweist:
a) Anbringen der Faser in dem Wurzelkanal in einer Weise, dass die Faser in direktem Kontakt mit der Behandlungsstelle ist; und
b) Halten der Faser an der Behandlungsstelle,
wodurch das Arzneimittel mit einer kontrollierten Geschwindigkeit der Behandlungsstelle zugeführt wird.

3. Verwendung nach Anspruch 1, bei der die endodontische Faser zur Verwendung in einem Behandlungsverfahren ist, das aufweist:
a) Einführen der Faser in einen gereinigten und gespülten Wurzelkanal in einer Weise, dass die Faser in direktem Kontakt mit der Behandtungs-Stelle ist; und
b) Halten der Faser an der Behandlungsstelle,
wodurch das Arzneimittel mit einer kontrollierten Geschwindigkeit der Behandlungsstelle verabreicht wird.

4. Verwendung nach Anspruch 3, bei der das Arzneimittel ein entzündungshemmendes Mittel ist und das Behandlungsverfahren in Schritt (a) das Anbringen der Faser in einem gereinigten und gespülten Wurzelkanal in einer Weise, dass die Faser in direktem Kontakt mit dem entzündeten Gewebe ist, beinhaltet; wodurch das entzündungshemmende Mittel der entzündeten Stelle zugeführt wird.

5. Verwendung nach einem der Ansprüche 1 bis 4, bei der die endodontische Faser ein Ethylenvinylacetat-Copolymer aufweist oder bei der die endodontische Faser einen Durchmesser von 0,1 bis 0,5 mm hat.

6. Verwendung nach einem der Ansprüche 1 bis 3, bei der das eine oder die mehreren Arzneimittel ausgewählt werden aus der Gruppe, die aus Antibiotika, antimikrobiellen Mitteln, entzündungshemmenden Mitteln, Immunreagentien und antigenbindenden Fragmenten besteht.

7. Verwendung nach einem der Ansprüche 1 bis 3, bei der das eine oder die mehreren Arzneimittel ein oder mehrere Antibiotika aufweisen, die ausgewählt werden aus Clindamycin, Tetracyclin, Neomycin, Kanamycin, Metranidazol und Canamycin.

8. Verwendung nach einem der Ansprüche 1 bis 3, bei der die Faser ein Ethylenvinylacetat-Copolymer mit einem Durchmesser von 0,1 bis 2,0 mm ist, und bei der das Arzneimittel Clindamycin ist, das in einer Dosis von 2,0 mg bis 5,0 mg pro 10 mm der Faser inkorporiert ist.

9. Verwendung nach einem der Ansprüche 1 bis 3, bei der das Copolymer-Vehikulum mit einem biologisch verträglichen kühlenden Spray behandelt wird.

## Revendications

1. Utilisation d'un ou plusieurs médicaments dans la fabrication d'une fibre endodontique pour la délivrance locale et la libération prolongée dudit (desdits) un ou plusieurs médicament(s) au niveau d'un site de traitement intracanal, dans laquelle la fibre endodontique comprend un véhicule comprenant un copolymère, au sein duquel le(s)dit(s) un ou plusieurs médicament(s) est(sont) incorporé (s) .

2. Utilisation selon la revendication 1, dans laquelle la fibre endodontique est destinée à une utilisation dans un procédé de traitement comprenant :
a) le positionnement de la fibre dans le canal radiculaire de sorte que la fibre soit en contact direct avec le site de traitement ; et
b) le maintien de la fibre au niveau du site de traitement,
ledit médicament étant délivré au niveau du site de traitement à une vitesse contrôlée.

3. Utilisation selon la revendication 1, dans laquelle la fibre endodontique est destinée à une utilisation dans un procédé de traitement comprenant :
a) l'insertion de la fibre dans un canal radiculaire débridé et irrigué de sorte que la fibre soit en contact direct avec le site de traitement ; et
b) le maintien de la fibre au niveau du site de traitement,
le médicament étant administré au niveau du site de traitement à une vitesse contrôlée.

4. utilisation selon la revendication 3, dans laquelle le médicament est un agent anti-inflammatoire, et ledit procédé de traitement implique dans l'étape (a) le positionnement de la fibre dans un canal radiculaire débridé et irrigué de sorte que la fibre soit en contact direct avec le tissu enflammé ; l'agent anti-inflammatoire étant délivré au niveau du site d'inflammation.

5. Utilisation selon l'une quelconque des revendications 1 à 4, dans laquelle la fibre endodontique comprend un copolymère d'acétate de vinyle et d'éthylène ou dans laquelle la fibre endodontique a un diamètre de 0,1 à 0,5 mm.

6. Utilisation selon l'une quelconque des revendications 1 à 3, dans laquelle le(s)dit(s) un ou plusieurs médicament(s) est(sont) choisi(s) dans le groupe constitué d'antibiotiques, d'agents antimicrobiens, d'agents anti-inflammatoires, d'immunoréactifs et de fragments de liaison à un antigène.

7. Utilisation selon l'une quelconque des revendications 1 à 3, dans laquelle le (s) dit (s) un ou plusieurs médicament (s) comprend (comprennent) un ou plusieurs antibiotique(s) choisi(s) parmi la clindamycine, la tétracycline, la néomycine, la kanamycine, le métranidazole et la canamycine.

8. Utilisation selon l'une quelconque des revendications 1 à 3, dans laquelle la fibre est un copolymère d'acétate de vinyle et d'éthylène, ayant un diamètre de 0,1 à 2,0 mm et dans laquelle ledit médicament est la clindamycine incorporée à une dose de 2,0 mg à 5,0 mg par 10 mm de fibre.

9. utilisation selon l'une quelconque des revendications 1 à 3, dans laquelle ledit véhicule comprenant un copolymère est traité avec un spray réfrigérant biocompatible.
